# EUROPEAN PATENT SPECIFICATION

(11) **EP 3 880 282 B1**
(45) Date of publication and mention of the grant of the patent: **01.03.2023**
(21) Application number: 20790050.7
(22) Date of filing: 11.09.2020
(51) Int. Cl.: A61M 16/04

(54) **TRACHEAL AID**
TRACHEALHILFE
DISPOSITIF TRACHÉAL

(30) Priority: 20.09.2019 IT 201900016781
(43) Date of publication of application: 22.09.2021
(73) Proprietor: Med Europe S.r.l., 40018 San Pietro in Casale (BO) (IT)
(72) Inventor: CHECCACCI CARBONI, Stefano, 35018 San Martino di Lupari (PD) (IT); BONALDI, Efrem, 37047 San Bonifacio (VR) (IT); GENNARO, Paolo, 35132 Padova (IT)
(74) Representative: Trentin, Michele
(86) International application number: PCT/IB2020/058466
(87) International publication number: WO 2021/053477

(56) References cited:
- EP-A1- 0 092 618
- WO-A1-2013/118059
- US-A- 4 351 330
- US-A- 5 507 284

## Description

### Definitions

In the present patent, the term "collapsible duct" means a duct free to collapse, making the walls touch. In such a configuration, the duct is to be understood as blocked.

### Field of application

The present invention is generally applicable to the medical field and relates to medical instruments for the management of patients' airways.

In particular, the present invention relates to a tracheal device usable for the assisted ventilation of patients, for example, under anaesthesia or with ongoing respiratory crises.

### State of the art

A number of situations are known in which people may go into respiratory crisis. Typically this occurs following trauma, but this phenomenon may also be induced, as in the case of patients who must undergo surgery involving the use of total anaesthesia.

In all such cases, the patient in respiratory crisis is rescued by means of assisted ventilation devices, usually represented by an Ambu balloon or ventilation machines.

For the functioning thereof, these devices must be able to access the lungs and this takes place by means of special tubes which cover the airways.

The most known type of tube is the endotracheal tube. They are typically made of plastic and are inserted into the patient's trachea, usually through the mouth and by using laryngoscopes.

These endotracheal tubes have a generally circular section and can have either a predefined and fixed shape or be flexible: the latter, for insertion, must be moulded through a rigid wire, called an obturator, removed once the insertion is completed.

The tube has in the proximal part, i.e., the part protruding from the mouth, a fitting to be connected to the artificial ventilation system. Furthermore, a small balloon called a cuff is arranged in the distal part of the tube, outside the tube itself. This balloon may be inflated from the outside, by an injection of air into a special duct, so as to adhere to the tracheal wall to prevent the entry of liquids into the lungs, prevent air from escaping and also to stabilize the tube in the correct place.

However, while safest for the patient, the endotracheal intubation procedure is difficult to apply. In fact, it requires being able to inspect the airways because blind entry means little, if any, chance of success. As is known, in fact, the access to the oesophagus is next to the trachea and this access is the most direct from both the mouth and the nose. An insertion without inspection therefore leads to very high possibilities of erroneous access to the oesophagus.

It follows that the intubation operation must be performed by experienced staff, with dedicated instruments and in safe facilities, all the more so since for at least a short time the patient is in a state of apnea.

Since the problems described have been found, in the case of urgent intervention on traumatized patients, the use of different tools known as supraglottic airway devices is used.

This is typically a tube fitted with an inflatable distal cuff and inserted into the supraglottic region. Therefore, its ease of application is due to the fact that it must not be inserted into the trachea; the insertion is easier as it does not require additional instruments.

However, this device does not protect the lungs from the risk of aspiration of fluid from the oesophagus by not blocking this passage. For this reason, supraglottic airway devices are used as an urgent intervention, but subsequently replaced with an endotracheal tube when the patient has been stabilized.

However, the application of the supraglottic airway device also requires experience on the part of the rescuer in order not to misplace it.

In addition, the replacement of the supraglottic airway device with endotracheal intubation at a later time is highly critical, since the patient is in apnea during the operation.

Often, however, it is convenient to insert medical accessories in the aforementioned orifices to support the intervention on the patient. For example, it is often useful to use a gastric tube for guided gastric regurgitation leakage. Other examples are the use of optical instruments for airway inspection or oesophageal inspection.

All these accessories are arranged parallel to the above-mentioned tubes and constitute an obstacle to the seal of the cuffs, both in the case of endotracheal devices and, above all, in the case of supraglottic airway devices.

In addition to the above, document WO 2013/118059 A1 is known, yet it also does not solve the drawbacks highlighted above.

### Presentation of the invention

The object of the present invention is to provide a tracheal device which allows at least partially overcoming the drawbacks highlighted above.

In particular, an object of the present invention is to provide a tracheal device which can be used as a supraglottic airway device or as an endotracheal tube without removing it from the position thereof and allowing the passage, also repeated, from one configuration to another.

Another object is that the tracheal device of the present invention allows to avoid periods of apnea for the patient even in the case of transition from use as a supraglottic airway device to use as an endotracheal tube and vice versa.

Another object of the present invention is to provide a tracheal device comprising a duct which allows the ejection of the regurgitated fluids from the stomach towards the outside of the patient. A further object of the present invention is that such a duct is, however, collapsible so as to minimize, if not eliminate, difficulties in ensuring an optimal seal of the orifices in moments of absence of regurgitation.

Another object of the present invention is to provide a tracheal device comprising a duct adapted to be covered by medical accessories such as, for example, gastric probes, obturators or optical instruments. A further object is that this duct is made of material adapted to optimize the sealing of the cuffs mentioned above, even in the presence of such accessories, whose manufacture typically involves the use of materials unsuitable for the purpose.

Another object of the present invention is to provide a tracheal device which can also be used successfully by inexperienced staff.

Said objects, as well as others which will become clearer below, are achieved by a tracheal device according to the following claims, which are to be considered as an integral part of the present patent.

In particular, it comprises at least a first tube having a proximal end positioned so as to be protruding from the patient's mouth and connectable to a device for assisted respiration. The distal end of the first tube may instead be usable to cover an orifice, typically consisting of the trachea or supraglottic region.

At least a first sealing element is externally coupled to said first tube to block the orifice into which the first tube may be inserted.

According to an aspect of the invention, the tracheal device also comprises at least a second tube having the distal end usable to cover an orifice, in this case typically consisting of the oesophagus. Similar to the first tube, a second sealing element is also externally coupled to the second tube to block the orifice into which the second tube may be inserted.

According to another aspect of the invention, the tracheal device also comprises at least one service duct, flexible and collapsible, stably coupled to the first and second tubes. This service duct allows to arrange the elements of the tracheal device according to at least two configurations: a first configuration in which the distal end of the first tube is inserted into the supraglottic region of the patient while the distal end of the second tube is inserted into the oesophagus of the patient, and a second configuration in which the distal end of the first tube is inserted into the trachea of the patient and the distal end of the second tube is inserted into the oesophagus of the patient.

The tracheal device of the invention, as will be seen in more detail, is therefore advantageously usable both as a supraglottic airway device and as an endotracheal tube, protecting the airway in both cases from regurgitation fluids coming from the stomach. In fact, if the user wants to use the device of the invention as a supraglottic airway device, the user can arrange the tubes in the first configuration, or the user can use the device as an endotracheal tube by arranging the tubes according to the second configuration.

The tracheal device of the invention advantageously also allows the passage from the first configuration to the second, thus allowing it to be transformed from a supraglottic airway device to an endotracheal tube and vice versa any number of times.

Still advantageously, since the transition from the configuration as supraglottic airway device to the configuration as endotracheal tube may be performed without extracting the tracheal device of the invention, the periods of apnea to which the patient is subjected are minimized, if not cancelled.

Still advantageously, the service duct, in addition to flexibly connecting the two tracheal tubes, puts the patient's oesophagus in fluid communication with the external environment, thus allowing the expulsion of the regurgitated materials from the stomach.

Still advantageously, being collapsible, the service duct minimizes, if not eliminates, the difficulties of blocking the orifices, allowing the sealing elements to adapt to the mucosal walls in which they are inserted, achieving an optimal fluidic seal.

Moreover, still advantageously, the service duct of the tracheal device is adapted to be covered by one or more medical accessories such as, for example, gastric probes, obturators or optical instruments. Still advantageously, this duct is made of material adapted to not hinder the blocking of the orifices guaranteed by the sealing elements even in the presence of such medical accessories.

### Brief description of the drawings

Further features and advantages of the invention will become more evident in light of the detailed description of a preferred but not exclusive embodiment of a tracheal device according to the invention, illustrated by way of non-limiting example with the aid of the accompanying drawings, in which:
FIG. 1 shows a tracheal device according to the invention;
FIGS. 2 and 3 show the tracheal device of FIG. 1 in different operating steps.

### Detailed description of some preferred embodiments

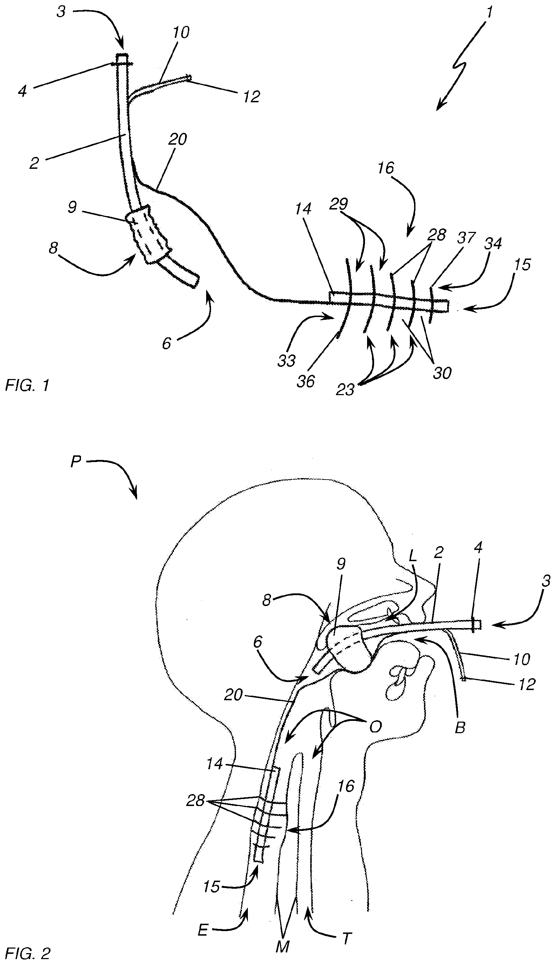

With reference to the above-mentioned figures, and in particular to figures 1 and 2, a tracheal device **1** according to the invention is described herein. It is adapted to be inserted by the mouth B into a patient P and comprises a first tube **2** having a proximal end **3** protruding from the mouth B of the patient **P** and connectable to a device for assisted ventilation. The latter is not the subject of the present patent and is not shown in the figures.

The proximal end 3 has a sealed fitting 4 connectable to the respirator for the passage of fluids for the respiration of the patient P.

The distal proximity 6 of the first tube 2, opposite the aforementioned proximal end 3, is usable to cover an orifice O such as, for example, the supraglottic region L or the trachea T.

According to an aspect of the invention, a sealing element 8 is present which is externally coupled to the aforementioned first tube **2.**

In the preferred embodiment of the invention, this sealing element **8** is a first cuff **9** connected to a pipe **10** terminating outside the patient **P** and also having a sealed fitting **12.** The latter can be used to inflate the cuff **9** using, for example, a syringe. Usually, the inflatable cuff **9** is arranged near the distal end **6** of the first tube **2.**

Obviously, this aspect should not be considered limiting for different embodiments of the invention, where, for example, the first sealing element does not comprise a cuff but one or more equivalent elements.

According to another aspect of the invention, the tracheal device **1** comprises a second tube **14** also having the distal end **15** usable for covering an orifice **O** which typically consists of the oesophagus **E.**

Typically, but not necessarily, both tubes **2** and **14** have a shape and size such that they can be inserted into the trachea **T,** however, as mentioned, for a correct operation of the device of the invention **1** the second tube **14** is inserted into the oesophagus **E.**

Typically, but not necessarily, the tubes **2** and **14** are cylindrical and made of biocompatible materials.

According to another aspect of the invention, the second tube **14** is also externally coupled to a second sealing element **16** which allows the oesophagus **E** to be blocked when the second tube **14** is inserted therein so as to avoid the passage of any gastric regurgitations.

According to a further aspect of the invention, the tracheal device **1** also comprises a service duct **20** stably coupled to the two tracheal tubes **2** and **14.** Typically, but not necessarily, this duct **20** is stably coupled to a portion of the first tube **2** identified between the first sealing element **8** and the proximal end **3.**

Furthermore, the duct **20** is flexible so as to allow the device **1** to be arranged according to two distinct configurations. There is a first configuration in which the distal end **6** of the first tube **2** is inserted into the supraglottic region **L** of the patient **P,** while the distal end **15** of the second tube **14** is inserted into the oesophagus **E.** In this configuration, the first sealing element **8,** which acts on the supraglottic region **L,** allows the tracheal device **1** to perform the function of a supraglottic airway device.

Instead in the second configuration, the distal end **6** of the first tube **2** is inserted into the trachea **T** of the patient **P,** while the distal end **15** of the second tube **14** is inserted into the oesophagus **E.** In this configuration, the first sealing element **8** allows the trachea **T** to be blocked, stabilizing the first tube **2,** while the second sealing element **16** allows the oesophagus **E** to be blocked, preventing the passage of regurgitations from the stomach. Consequently, in such a configuration the tracheal device **1** of the invention performs the function of an endotracheal tube.

Advantageously, therefore, the service duct **20** always allows to arrange the tracheal device **1** so as to ensure the ventilation of the patient.

The **tracheal device 1** allows, still advantageously, to be used both as a supraglottic airway device (first configuration) and as an endotracheal tube (second configuration). Moreover, the structure thereof allows to switch from one configuration to another reversibly and without extracting the device **1** from the patient **P.**

Still advantageously, since the transition from the configuration as supraglottic airway device to the configuration as endotracheal tube may be performed without extracting the tracheal device **1,** the periods of apnea to which the patient **P** is subjected are minimized, if not cancelled.

In addition to this, the service duct **20** is collapsible, i.e., free to collapse, making the walls touch so as to be blocked.

Advantageously, therefore, the service duct **20** minimizes, if not eliminates, the difficulties of blocking the orifices **O,** allowing the sealing elements **8** and **16** to adapt to the mucosal walls **M** in which they are inserted, achieving an optimal fluidic seal. Moreover, the same service duct **20** is typically made with material such as to reduce hindering the blocking mentioned above, even if it is not collapsed.

Still advantageously, the service duct **20** puts the oesophagus **E** of the patient **P** in fluidic communication with the external environment. This duct **20** is typically hollow and therefore advantageously allows the expulsion of the fluids regurgitated by the stomach due to the pressure exerted by the latter (passive expulsion). In fact, this pressure allows to reverse the collapse process of the duct **20,** opening it.

Obviously, this aspect should not be considered limiting for different embodiments of the invention, where, for example, the tracheal device comprises multiple service ducts.

According to another aspect of the invention not shown in the figures, the tracheal device **1** of the invention also comprises one or more medical accessories capable of covering the service duct **20.** These accessories belong to the group comprising gastric probes, obturators and optical instruments. However, they can be used simultaneously in the aforementioned case of the presence of several service ducts.

Advantageously, such medical accessories make it possible to render the service duct **20** active. In this sense, it can be used to provide for the outflow of regurgitated fluids and the artificial nutrition of patients by covering it with a gastric tube, or it can be used to facilitate the insertion of the **tracheal device 1** in the patient by covering it with an obturator. Further, it may be covered by optical instruments for visual inspection of the orifices **O.** In all such cases, still advantageously, the service duct **20** allows the use of the medical accessories without problems of operation of the sealing elements **8** and **16** as well as without problems of compatibility with the mucosal walls **M** of the accessories since they do not come into contact therewith.

With regard to the second sealing element **16,** according to the described embodiment, it comprises several elastically deformable bodies **23** arranged wrapping the second tube **14** to be interposed between the same and the mucosal walls **M.** In this way they create a fluidic seal through the deformation thereof. The number of deformable bodies should not be considered limiting for different embodiments of the invention where, for example, there is only one.

Advantageously, the deformable bodies **16** wrapping the second tube **14,** being adaptable to the mucosal walls **M,** allow an optimal blocking of the oesophageal orifice **O.**

In the embodiment described, the deformable bodies **23** comprise several discoid elements **28** arranged radially to the second tube **14** and between which material vacancy areas **29** are interposed.

Still advantageously, the fluidic seal of the oesophagus **E** is obtained without any intervention of the staff using the tracheal device **1.** Moreover, if the second sealing element consists of an inflatable cuff, an important aspect is that it must not be inflated excessively in order to avoid damage to the mucosal walls. According to the embodiment shown in the figures, this is instead advantageously excluded. The possibility of human error is therefore excluded, especially in the case of use by inexperienced or hardly experienced staff.

Obviously, the manufacture of the sealing element is a feature which should not be understood in a limiting sense for the present invention. In particular, an alternative embodiment where the second sealing element comprises a second inflatable cuff connected to at least a second pipe for the passage of inflation fluid is not excluded. According to this embodiment, not shown in the figures, this second duct can be coupled externally to the service duct or embedded in the walls thereof.

Further, according to different embodiments not shown in the figures, the deformable bodies are formed by a single body shaped as a full ellipsoid or, according to further different embodiments also not represented herein, by a plurality of full ellipsoid or spherical bodies.

In the drawings it can be seen that, according to the embodiment represented therein, the discoid elements **23** have increasing extensions starting from the distal end **15** of the second tube **14.** Advantageously, this allows to obtain a progressive fluidic seal which is well suited to different oesophageal dimensions from patient to patient without the risk of subjecting the mucosal walls **M** to excessive pressure.

Obviously, this aspect should not be considered limiting for different embodiments of the invention, where, for example, the discoid elements are of any extension, of different forms or all equal to each other.

With regard to the material vacancy areas **29,** the contact of the discoid elements **28** with the mucosal walls **M** allows to obtain a succession of decompression chambers **30** which allow an outlet for the regurgitation of fluids coming from the stomach while preventing the access thereof to the airways.

According to another aspect of the invention, each of the deformable bodies **23** comprises a casing **33** which hermetically seals an inner portion thereof **34** so as to render it inaccessible from the outside. In such a case, as specified above, it does not need to be inflated or filled in the operating step with any solid, liquid or gas.

Advantageously, the aforementioned shape allows to differentiate the materials of the casing **33** and the inner portion **34** so as to optimize the function thereof. In particular, the casing **33** must correctly adhere to the mucosal walls **M** and the choice of material allows this property to be optimized while limiting the use of biocompatible materials. At the same time, materials more adapted for obtaining the desired pressure on the mucosal walls **M** may be used for the inner portion. Moreover, advantageously, this configuration makes it possible to obtain the inner portion **34** also with materials in the liquid or gaseous state.

Obviously, this aspect should not be considered limiting for different embodiments of the invention, where the deformable bodies are obtained in a single body and with a single material.

In the case of the embodiment of the invention shown in the figures, in the discoid elements **28** the casing **33** is formed by the outer surface **36** of each discoid element **28,** while the inner portion **34,** is formed by the thickness **37** thereof.

According to a further aspect of the invention, not visible in the figures, the second tube **14** comprises two or more lumens, at least one of which is fluidly coupled to the service duct **20.**

Advantageously, the presence of two or more lumens allows to be able to make them adapted to be covered by accessory devices such as, for example, optical instruments or the like.

Obviously, this aspect should not be considered limiting for different embodiments of the invention, where, for example, the second tube comprises a single lumen.

According to different embodiments of the invention, not shown herein, the tracheal device comprises a further medical accessory, i.e., a semi-rigid stylet shaped to cover the two tubes. Said stylet is removable and the functionality thereof is to keep the tubes mutually aligned during the insertion thereof into the patient. After insertion, the stylet is extracted so as to leave the flexible junction element freedom of action.

Moreover, according to a further embodiment not shown in the figures, the stylet may be coupled to additional medical devices useful for the insertion thereof, such as, for example, instruments provided with final optics.

Advantageously, the presence of an optical detection instrument allows the user to guide the insertion of the tracheal device with the possibility of seeing the orifice being covered.

According to further embodiments, however, such auxiliary medical devices, and in particular the imaging means, are coupled to the distal end of the second tube or to different parts thereof.

According to another embodiment, the metal stylet has a tip which is steerable by means of a cable. Advantageously, in this manner the user can improve the steering of the device in the insertion into the patient's orifices.

According to a further embodiment, the tracheal device also comprises an inflatable blister interposed between the second tube and the elastically deformable bodies. This blister can be inflated as needed by the operator through a pipe.

Advantageously, the inflation of said blister exerts a pressure on the inner parts of the deformable bodies so as to cause the slight expansion thereof, which results in an increase in the pressure they exert against the mucosal walls. This allows to further stabilize the fluidic seal of the oesophageal device.

Operationally, the user inserts the tracheal device **1** of the invention into the mouth **B** of the patient **P.** In the absence of airway inspection ("blind" insertion), the second tube **14** most likely covers the oesophagus **E,** as shown in fig. 2. It follows that with this operation the discoid elements **28,** interposed between the mucosal walls **M** and the second tube **14,** block the oesophagus **E,** obtaining a fluidic seal which preserves the airways from any regurgitated fluids. Moreover, these can be evacuated through the service duct **20** passively capable of dilating.

In the case in which the user wishes to use the device **1** of the invention as a supraglottic airway device, the user continues with the insertion until the first cuff **9** associated with the first tube **2** reaches the supraglottic region **L.** Subsequently, the user can proceed with the inflation of the cuff **9,** blocking the supraglottic region **L.**

The user can then connect the assisted respiration device to the sealed fitting **4** present at the proximal end **3** of the first tube **2,** successfully ventilating the patient **P.**

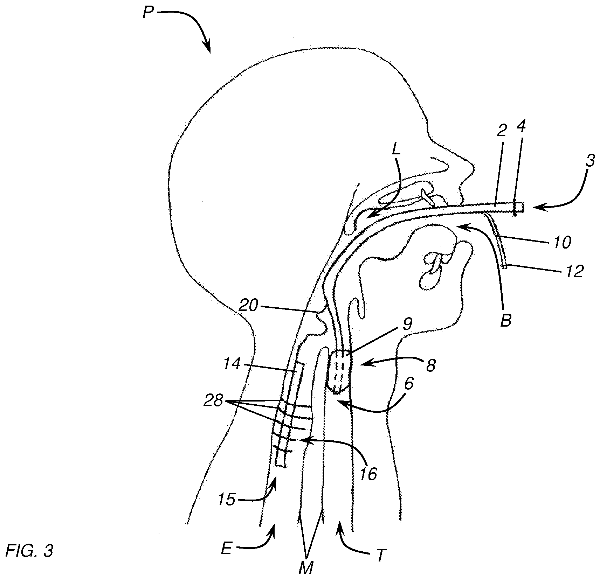

If it is necessary to proceed, also later, with the intubation of the patient **P,** passing from the supraglottic airway device configuration to an endotracheal tube configuration, it is not necessary to remove the device **1.** In fact, it is sufficient to proceed, as shown in fig. 3, to insert the distal end **6** of the first tube **2** into the trachea **T.** This operation can advantageously be carried out without removing the second tube **14** thanks to the flexibility of the service duct **20** which allows the distal end **6** of the first tube **2** to cover a different orifice **O** than the one covered by the second tube **14.**

Advantageously, accordingly, it is demonstrated that the **tracheal device 1** of the invention can be used both as a supraglottic airway device and as an endotracheal tube.

Still advantageously, such transformation from one configuration to another is reversible any number of times.

In addition, still advantageously, since the transition from the configuration as supraglottic airway device to the configuration as endotracheal tube may be performed without extracting the tracheal device **1** of the invention, the periods of apnea to which the patient is subjected are minimized, if not cancelled.

Typically, but not necessarily, the transformation from supraglottic airway device to tracheal tube operatively takes place by first inserting a fibroscope into the first tube **2** to locate the trachea **T,** then the trachea **T** is covered with the fibroscope, then the cuff **9** is deflated, still in the supraglottic region **L,** and finally the first tube **2** is inserted into the trachea **T** using the fibroscope as a guide. Obviously, the use of the fibroscope is optional and this procedure should not be considered limiting for the present invention since the same operation can be carried out with a more traditional laryngoscopy.

Following insertion into the trachea **T,** the cuff **9** is inflated, ending the transformation of the device **1** of the invention from supraglottic airway device to endotracheal tube.

What emerges from all the above is that the tracheal device **1** of the invention can always be used "blindly", that is, in the absence of airway inspection without jeopardizing the failure of the application of assisted respiration to the patient **P.** In addition, this operation can advantageously be carried out by inexperienced staff and also in critical situations of the patient **P.**

In light of the above, it is to be understood that the tracheal device of the invention overcomes the drawbacks of the known art, being easy to apply on any patient in any state they may be found.

In particular, it can also be used successfully by inexperienced staff, allowing the use of a single product capable of being configured as a supraglottic airway device and endotracheal tube, avoiding replacement/extraction procedures to make the transition from one to the other, also repeated.

The device of the invention also allows to make the transition from one configuration to another in a reversible manner and limiting, if not cancelling, the time in which the patient is in an apnea condition.

Furthermore, the device of the invention comprises a collapsible service duct which, in addition to putting the oesophagus in fluidic communication with the external environment to expel the regurgitation fluids coming from the stomach, allows the sealing elements to adapt to the mucosal walls of the orifices in which the device is inserted, achieving optimal blocking.

The aforementioned duct is then adapted to be covered by multiple medical accessories, for example gastric probes, obturators or optical instruments.

In addition, the tracheal device of the invention is shaped to block the oesophagus by progressively adapting to the mucosal walls of the orifice, so as to prevent regurgitated fluids from reaching the airway.

Finally, the tracheal device of the invention, adapted for use both in the supraglottic airway device configuration and as a tracheal tube, is configured to be inserted "blindly", making it suitable for use even by inexperienced staff.

The invention might be subject to many changes and variants, which are all included in the appended claims. Moreover, all the details and steps may furthermore be replaced by other technically equivalent elements, and the materials may be different depending on the needs, without departing from the protection scope of the invention defined by the appended claims.

## Claims

1. A tracheal device usable for the assisted respiration of a patient (**P**), comprising:
- at least a first tube (**2**), having a proximal end (**3**) to be arranged protruding from the mouth (**B**) of the patient (**P**) and connectable to an assisted respiration device, and a distal end (**6**) usable to cover an orifice (**O**);
- at least a first sealing element (**8**) externally coupled to said first tube (**2**);
- at least a second tube (**14**) having the distal end (**15)** usable to cover an orifice (**O**);
- at least a second sealing element (**16**) externally coupled to said second tube (**14**);
said tracheal device (**1**) being **characterized in** comprising at least one flexible and collapsible service duct (**20**) stably coupled to said first tube (**2**) and said second tube (**14**) to allow said duct (**20**) and said tubes (**2, 14**) to be arranged in a first configuration in which said distal end (**6**) of said first tube (**2**) is inserted into the supraglottic region (**L**) of the patient (**P**) and said distal end (**15**) of said second tube (**14**) is inserted into the oesophagus (**E**) of the patient (**P**), and in a second configuration in which said distal end (**6**) of said first tube (**2**) is inserted into the trachea (**T**) of the patient (**P**) and said distal end (**15**) of said second tube (**14)** is inserted into the oesophagus (**E**) of the patient (**P**).

2. Tracheal device according to claim 1, **characterized in that** said service duct (**20)** is stably coupled to a portion of said first tube (**2**) identified between said first sealing element (**8**) and said proximal end (**6**).

3. Tracheal device according to claims 1 or 2, comprising one or more medical accessories capable of covering said service duct (**20)**.

4. Tracheal device according to claim 3, **characterized in that** said one or more medical accessories belong to the group comprising gastric probes, obturators and optical instruments.

5. Tracheal device according to any one of the preceding claims, **characterized in that** said first sealing element (**8**) is a first inflatable cuff (**9**) connected to at least one first duct (**10**) for the passage of inflation fluid.

6. Tracheal device according to any one of the preceding claims, **characterized in that** said second sealing element comprises a second inflatable cuff connected to at least a second duct for the passage of inflation fluid.

7. Tracheal device according to any one of claims 1 to 5, **characterized in that** said second sealing element (**16**) comprises one or more elastically deformable bodies (**23**) arranged wrapping said second tube (**14**) to be interposed between said second tube (**14**) and one or more mucosal walls (**M**) creating, by the deformation thereof, a fluid seal therewith, each of said one or more elastically deformable bodies (**23**) comprising a casing (**33**) which hermetically closes an inner portion (**34**) so as to make it inaccessible from the outside.

8. Tracheal device according to claim 7, **characterized in that** said one or more elastically deformable bodies (**23**) comprise several discoid elements (**28**) arranged radially to said second tube (**14**) and between which material vacancy areas (**29**) are interposed, said casing (**33**) being formed by an outer surface (**36**) of each of said discoid elements (**28**) and said inner portion (**34**) being formed by a thickness (**37**) of said discoid elements (**28**).

9. Tracheal device according to claim 7 or 8, comprising an inflatable blister interposed between said second tube and said one or more elastically deformable bodies.

10. Tracheal device according to any one of the preceding claims, **characterized in that** said second tube comprises two or more lumens, at least one of which is fluidly coupled to said service duct.

## Patentansprüche

1. Trachealvorrichtung, die für die assistierte Beatmung eines Patienten (**P**) verwendbar ist, umfassend:
- mindestens einen ersten Tubus (**2**) mit einem proximalen Ende (**3**), das aus dem Mund (**B**) des Patienten (**P**) vorstehend anzuordnen ist und mit einem Gerät zur assistierten Beatmung verbunden werden kann, und einem distalen Ende (**6**), das verwendbar ist, um eine Öffnung (**O**) zu bedecken;
- mindestens ein erstes Dichtungselement (**8**), das extern an den ersten Tubus (**2**) gekoppelt ist;
- mindestens einen zweiten Tubus (**14**) mit einem distalen Ende (**15**), das verwendbar ist, um eine Öffnung (**O**) zu bedecken;
- mindestens ein zweites Dichtungselement (**16**), das extern an den zweiten Tubus (**14**) gekoppelt ist;
wobei die Trachealvorrichtung (**1**) **dadurch gekennzeichnet ist, dass** sie mindestens einen flexiblen und zusammenfaltbaren Versorgungskanal (**20)** umfasst, der stabil an den ersten Tubus (**2**) und den zweiten Tubus (**14**) gekoppelt ist, um dem Kanal (**20**) und den Tuben (**2**, **14**) zu ermöglichen, in einer ersten Konfiguration, in der das distale Ende (**6**) des ersten Tubus (**2**) in die supraglottische Region (**L**) des Patienten (**P**) eingesetzt ist und das distale Ende (**15**) des zweiten Tubus (**14**) in die Speiseröhre (**E**) des Patienten (**P**) eingesetzt ist, und in einer zweiten Konfiguration, in der das distale Ende (**6**) des ersten Tubus (**2**) in die Luftröhre (**T**) des Patienten (**P**) eingesetzt ist und das distale Ende (**15**) des zweiten Tubus (**14**) in die Speiseröhre (**E**) des Patienten (**P**) eingesetzt ist, angeordnet zu werden.

2. Trachealvorrichtung nach Anspruch 1, **dadurch gekennzeichnet, dass** der Versorgungskanal (**20**) stabil an einen Abschnitt des ersten Tubus (**2**) gekoppelt ist, der zwischen dem ersten Dichtungselement (**8**) und dem proximalen Ende (**6**) definiert ist.

3. Trachealvorrichtung nach Anspruch 1 oder 2, umfassend ein oder mehrere medizinische Zubehörteile, die imstande sind, den Versorgungskanal (**20**) zu bedecken.

4. Trachealvorrichtung nach Anspruch 3, **dadurch gekennzeichnet, dass** das eine oder die mehreren medizinischen Zubehörteile zu der Gruppe gehören, die Magensonden, Obturatoren und optische Instrumente umfasst.

5. Trachealvorrichtung nach einem der vorstehenden Ansprüche, **dadurch gekennzeichnet, dass** das erste Dichtungselement (**8**) eine erste aufblasbare Manschette (**9**) ist, die mit mindestens einem ersten Kanal (**10)** für den Durchgang von Aufblasfluid verbunden ist.

6. Trachealvorrichtung nach einem der vorstehenden Ansprüche, **dadurch gekennzeichnet, dass** das zweite Dichtungselement eine zweite aufblasbare Manschette umfasst, die mit mindestens einem zweiten Kanal für den Durchgang von Aufblasfluid verbunden ist.

7. Trachealvorrichtung nach einem der Ansprüche 1 bis 5, **dadurch gekennzeichnet, dass** das zweite Dichtungselement (**16**) einen oder mehrere elastisch verformbare Körper (**23**) umfasst, die den zweiten Tubus (**14**) umhüllend angeordnet sind, um zwischen dem zweiten Tubus (**14**) und einer oder mehreren Schleimhautwänden (**M**) eingefügt zu sein, wodurch, durch deren Verformung, eine Fluiddichtung damit erzeugt wird, wobei jeder des einen oder der mehreren elastisch verformbaren Körper (s) (**23**) ein Gehäuse (**33**) umfasst, das einen inneren Abschnitt (**34**) hermetisch verschließt, um diesen von außen unzugänglich zu machen.

8. Trachealvorrichtung nach Anspruch 7, **dadurch gekennzeichnet, dass** der eine oder die mehreren elastisch verformbaren Körper (**23**) mehrere scheibenförmige Elemente (**28**) umfassen, die radial an dem zweiten Tubus (**14**) angeordnet sind und zwischen welchen materialfreie Flächen (**29**) eingefügt sind, wobei das Gehäuse (**33**) durch eine Außenfläche (**36**) jedes der scheibenförmigen Elemente (**28**) gebildet wird und der innere Abschnitt (**34**) durch eine Dicke (**37**) der scheibenförmigen Elemente (**28**) gebildet wird.

9. Trachealvorrichtung nach Anspruch 7 oder 8, umfassend eine aufblasbare Blase, die zwischen dem zweiten Tubus und dem einen oder den mehreren elastisch verformbaren Körper(n) (**23**) eingefügt ist.

10. Trachealvorrichtung nach einem der vorstehenden Ansprüche, **dadurch gekennzeichnet, dass** der zweite Tubus zwei oder mehr Lumen umfasst, von welchen mindestens eines strömungstechnisch an den Versorgungskanal gekoppelt ist.

## Revendications

1. Dispositif trachéal utilisable pour la respiration assistée d'un patient (**P**), comprenant :
- au moins un premier tube (**2**), présentant une extrémité proximale (**3**) à agencer de manière à dépasser de la bouche (**B**) du patient (**P**) et pouvant être raccordée à un dispositif de respiration assistée, et une extrémité distale (**6**) utilisable pour recouvrir un orifice (**O**) ;
- au moins un premier élément d'étanchéité (**8**) couplé en externe audit premier tube (**2**) ;
- au moins un second tube (**14**) présentant l'extrémité distale (**15**) utilisable pour recouvrir un orifice (**O**) ;
- au moins un second élément d'étanchéité (**16**) couplé en externe audit second tube (**14**) ;
ledit dispositif trachéal (**1**) étant **caractérisé en ce qu'il** comprend au moins un conduit de service souple et pliable (**20**) couplé de manière stable audit premier tube (**2**) et audit second tube (**14**) pour permettre audit conduit (**20**) et auxdits tubes (**2, 14**) d'être agencés dans une première configuration dans laquelle ladite extrémité distale (**6**) dudit premier tube (**2**) est insérée dans la région supraglottique (**L**) du patient (**P**) et ladite extrémité distale (**15**) dudit second tube (**14**) est insérée dans l'œsophage (**E**) du patient (**P**), et dans une seconde configuration dans laquelle ladite extrémité distale (**6**) dudit premier tube (**2**) est insérée dans la trachée (**T**) du patient (**P**) et ladite extrémité distale (**15**) dudit second tube (**14**) est insérée dans l'œsophage (**E**) du patient (**P**).

2. Dispositif trachéal selon la revendication 1, **caractérisé en ce que** ledit conduit de service (**20**) est couplé de manière stable à une portion dudit premier tube (**2**) identifiée entre ledit premier élément d'étanchéité (**8**) et ladite extrémité proximale (**6**).

3. Dispositif trachéal selon les revendications 1 ou 1, comprenant un ou plusieurs accessoires médicaux capables de recouvrir ledit conduit de service (**20**).

4. Dispositif trachéal selon la revendication 3, **caractérisé en ce que** lesdits un ou plusieurs accessoires médicaux appartiennent au groupe comprenant des sondes gastriques, des obturateurs et des instruments optiques.

5. Dispositif trachéal selon l'une quelconque des revendications précédentes, **caractérisé en ce que** ledit premier élément d'étanchéité (8) est un premier brassard gonflable (9) raccordé à au moins un premier conduit (10) pour le passage de fluide de gonflage.

6. Dispositif trachéal selon l'une quelconque des revendications précédentes, **caractérisé en ce que** ledit second élément d'étanchéité comprend un second brassard gonflable raccordé à au moins un second conduit pour le passage de fluide de gonflage.

7. Dispositif trachéal selon l'une quelconque des revendications 1 à 5, **caractérisé en ce que** ledit second élément d'étanchéité (**16**) comprend un ou plusieurs corps élastiquement déformables (**23**) agencés de manière à envelopper ledit second tube (**14**) à interposer entre ledit second tube (**14**) et une ou plusieurs parois muqueuses (**M**) créant, par la déformation de ceux-ci, un joint fluide avec celles-ci, chacun desdits un ou plusieurs corps élastiquement déformables (**23**) comprenant un boîtier (**33**) qui ferme hermétiquement une portion intérieure (**34**) de manière à la rendre inaccessible depuis l'extérieur.

8. Dispositif trachéal selon la revendication 7, **caractérisé en ce que** lesdits un ou plusieurs corps élastiquement déformables (**23**) comprennent plusieurs éléments discoïdes (**28**) agencés radialement par rapport audit second tube (**14**) et entre lesquels des zones de lacune de matériau (**29**) sont interposées, ledit boîtier (**33**) étant formé par une surface extérieure (**36**) de chacun desdits éléments discoïdes (**28**) et ladite portion intérieure (**34**) étant formée par une épaisseur (**37**) desdits éléments discoïdes (**28**).

9. Dispositif trachéal selon la revendication 7 ou 8, comprenant une vésicule gonflable interposée entre ledit second tube et lesdits un ou plusieurs corps élastiquement déformables.

10. Dispositif trachéal selon l'une quelconque des revendications précédentes, **caractérisé en ce que** ledit second tube comprend deux lumières ou plus, dont au moins une est fluidiquement couplée audit conduit de service.
